(19) Europäisches Patentamt
European Patent Office
Office européen des brevets

(11) **EP 4 382 901 A1**

(12) **EUROPEAN PATENT APPLICATION**
published in accordance with Art. 153(4) EPC

(43) Date of publication:
**12.06.2024 Bulletin 2024/24**

(21) Application number: **22852650.5**

(22) Date of filing: **31.05.2022**

(51) International Patent Classification (IPC):
***G01N 27/416*** (2006.01)

(52) Cooperative Patent Classification (CPC):
**G01N 27/416**

(86) International application number:
**PCT/JP2022/022023**

(87) International publication number:
**WO 2023/013222 (09.02.2023 Gazette 2023/06)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **03.08.2021 JP 2021127816**

(71) Applicant: HITACHI HIGH-TECH CORPORATION
**Tokyo 105-6409 (JP)**

(72) Inventors:
• **Rahma Hutami Rahayu**
**Tokyo 105-6409 (JP)**
• **MIYAKE Masafumi**
**Tokyo 105-6409 (JP)**
• **YAMAMOTO Haruyoshi**
**Tokyo 105-6409 (JP)**

(74) Representative: **MERH-IP Matias Erny Reichl
Hoffmann
Patentanwälte PartG mbB
Paul-Heyse-Strasse 29
80336 München (DE)**

(54) **ELECTROLYTE ANALYSIS APPARATUS AND ANALYSIS METHOD**

(57) Provided are an electrolyte analyzer and an analysis method capable of reducing a burden related to measurement while preventing a decrease in the accuracy of measurement results.

The electrolyte analyzer includes an electrolyte analysis unit including an ion-selective electrode used for measuring a specific ion, and a reference electrode; a diluent supply unit that supplies a diluent for diluting a reagent or a sample; and a control device, and measures the ion concentration in a liquid, in which the control device measures each of the diluted reagents with known specific ion concentrations at multiple different concentrations generated by diluting the reagents with known concentrations with a diluent, and performs a water quality determination process of the diluent based on the inclination obtained from the measurement results.

[FIG. 2]

EP 4 382 901 A1

EP 4 382 901 A1

**Description**

Technical Field

The present invention relates to an electrolyte analyzer and an analysis method.

Background Art

An electrolyte analyzer is a device that measures a specific electrolyte concentration contained in an electrolyte solution (hereinafter, may be referred to as a sample) such as blood or urine of a human body. For example, the electrolyte analyzer uses an ion-selective electrode method in which an ion-selective electrode is used to measure a concentration. For example, a flow type electrolyte analyzer is known as the electrolyte analyzer. A sample solution obtained by directly diluting serum serving as an electrolyte solution or diluting serum with a sample diluent is supplied to the ion-selective electrode in the flow type electrolyte analyzer, and the flow type electrolyte analyzer measures a liquid potential between the ion-selective electrode and a reference electrode solution. Subsequently (or prior to the measurement), a standard solution having a known electrolyte concentration is supplied to the ion-selective electrode, and a liquid potential between the standard solution and the reference electrode solution is measured in the same manner as the serum (or a sample solution). Then, an electrolyte concentration of the serum (or the sample solution) can be calculated based on the two liquid potentials of the liquid potential between the standard solution and the reference electrode solution and the liquid potential between the serum (or the sample solution) and the reference electrode solution.

As a measurement method using such an electrolyte analyzer, for example, PTL 1 discloses a method for measuring a concentration of chlorine ions, in which a chlorine ion-selective electrode and a reference electrode are immersed in a liquid to be measured, and a silver or silver chloride electrode is used as the chlorine ion-selective electrode and the silver chloride is dissolved in a saturated concentration in a solution in contact with the electrode in order to measure a concentration of the chlorine ions in the liquid to be measured based on a potential difference between the chlorine ion-selective electrode and the reference electrode.

PTL 2 discloses an electrolyte concentration measurement device including a drug substance supply unit that supplies a drug substance corresponding to a reagent accommodated in a bottle, a pure water supply unit that supplies pure water to the bottle, and a stirring mechanism that stirs and mixes the drug substance and the pure water in the bottle in which the drug substance is supplied from the drug substance supply unit and the pure water is supplied from the pure water supply unit.

Citation List

Patent Literature

PTL 1: JPH8-211016A
PTL 2: JP2018-17543A

Summary of Invention

Technical Problem

In the technique disclosed in PTL 1, a silver nitrate solution (AgNOS) is used as a diluent. However, when the silver nitrate solution is used as the diluent, wastewater treatment is indispensable in consideration of an environmental load, and the wastewater treatment causes an increase in treatment cost and treatment time. In particular in a large hospital, a clinical examination center, or the like that performs a large number of measurement processes, since a usage amount of a diluent and an amount of wastewater are large, a burden of running cost or the like is enormous.

In the technique disclosed in PTL 2, a diluent is produced by mixing a diluent stock solution and pure water. However, wastewater treatment is also indispensable in this case in consideration of an environmental load. In addition, although pure water is used for dilution of the stock solution, since pure water is not standardized in the field of clinical examination, an error may occur in a measurement result of the electrolyte analyzer due to unspecified ions contained in the pure water.

The invention has been made in view of the above problems, and an object of the invention is to provide an electrolyte analyzer and an analysis method that are capable of reducing a burden related to measurement while preventing a decrease in accuracy of a measurement result.

Solution to Problem

The present application includes a plurality of devices for solving the above problems. For example, an electrolyte analyzer includes: an electrolyte analysis unit including an ion-selective electrode used for measuring a specific ion, and a reference electrode; a diluent supply unit that supplies a diluent for diluting a reagent or a sample; and a control device, and the electrolyte analyzer measures an ion concentration in a liquid. The control device measures each of diluted reagents with known specific ion concentrations at multiple different concentrations generated by diluting the reagents with known concentrations with the diluent, and performs a water quality determination process of the diluent based on an inclination obtained from a measurement result.

Advantageous Effects of Invention

A burden related to measurement can be reduced while a decrease in accuracy of a measurement result can be prevented.

Brief Description of Drawings

[FIG. 1] FIG. 1 is a diagram schematically showing an overall configuration of an electrolyte analyzer according to a first embodiment.
[FIG. 2] FIG. 2 is a diagram showing the relationship between the potential difference between an ion-selective electrode and a reference electrode, and the concentration of the ions to be measured, when the system water in which the ions to be measured of the ion-selective electrode are dissolved is measured by the electrolyte analyzer.
[FIG. 3] FIG. 3 is a flowchart showing a water quality determination process according to the first embodiment.
[FIG. 4] FIG. 4 is a flowchart showing a water quality determination process according to a second embodiment.
[FIG. 5] FIG. 5 is a flowchart showing a water quality determination process according to a third embodiment.

Description of Embodiments

Hereinafter, embodiments of the invention will be described with reference to the drawings.

<First Embodiment>

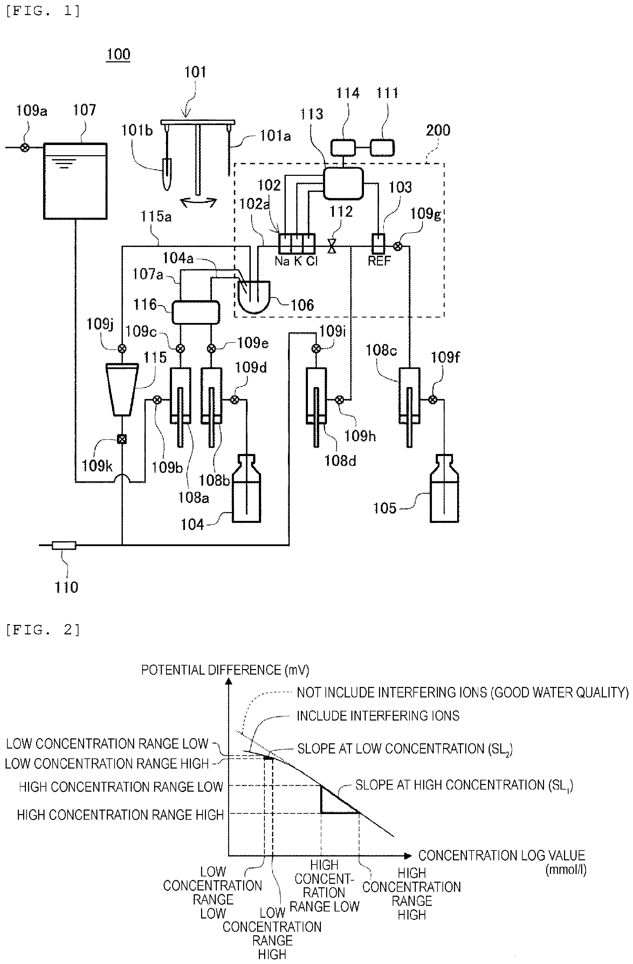

A first embodiment of the invention will be described in detail with reference to FIGS. 1 to 3.
FIG. 1 is a diagram schematically showing an overall configuration of an electrolyte analyzer according to an embodiment of the invention. A flow type electrolyte analyzer (hereinafter, simply referred to as an electrolyte analyzer) using an ion-selective electrode (ISE) will be described as an example of the electrolyte analyzer according to the embodiment.
In FIG. 1, an electrolyte analyzer 100 schematically includes a sample dispensing device 101, an ion-selective electrode group 102, a reference electrode 103, an internal standard solution bottle 104, a reference electrode solution bottle 105, a dilution tank 106, a water supply tank 107, syringes 108a to 108d, valves 109a to 109k, a waste liquid tank 110, and a control device 111.
The sample dispensing device 101 includes a sample probe 101a and a sample container 101b. The sample probe 101a performs a dispensing process of aspirating a sample (such as a sample of a patient) held in the sample container 101b and discharging the sample into the dilution tank 106. Here, the sample is a generic term of analysis targets collected from a living body of a patient, and is, for example, blood or urine. The sample may be a sample obtained by performing a preprocess on the analysis targets.
The water supply tank 107 stores highly-pure water (hereinafter, referred to as system water) that is generally used in the electrolyte analyzer. The system water is supplied from a pure water production device (not shown) or the like to the water supply tank 107 via the valve 109a. The system water stored in the water supply tank 107 is discharged to the dilution tank 106 via a system water nozzle 107a by operations of the syringe (a system water syringe) 108a and the valves 109b and 109c.
The internal standard solution bottle 104 accommodates a standard solution having a predetermined known ion concentration for an ion species to be measured by the electrolyte analyzer 100. An internal standard solution accommodated in the internal standard solution bottle 104 is generated in a manner of containing, for example, sodium ions ($Na+$), potassium ions ($K+$), and chlorine ions ($C1-$). The internal standard solution accommodated in the internal standard solution bottle 104 is discharged to the dilution tank 106 via an internal standard solution nozzle 104a by operations of the syringe (an internal standard solution syringe) 108b and the valves 109d and 109e.

A temperature controller 116 is provided in flow paths of the system water nozzle 107a and the internal standard solution nozzle 104a, and a temperature of the system water or the internal standard solution passing through the flow paths is adjusted to fall within a predetermined constant temperature range.

The reference electrode solution bottle 105 accommodates a reference electrode solution having a predetermined known ion concentration. The reference electrode solution accommodated in the reference electrode solution bottle 105 is, for example, an aqueous solution of potassium chloride (KCl) containing potassium ions (K+) and chlorine ions (Cl-). The reference electrode solution accommodated in the reference electrode solution bottle 105 is sent to the reference electrode 103 by operations of the syringe (a reference electrode solution syringe) 108c and the valves 109f and 109g.

The sample dispensed into the dilution tank 106 is diluted by the system water discharged from the water supply tank 107 to the dilution tank 106 via the system water nozzle 107a by operations of the syringe (a system water syringe) 108a and the valves 109b and 109c, and is stirred. Although the system water is used as a diluent for diluting the sample in the present embodiment, other diluents may be used to dilute the sample. The sample diluted with the system water in the dilution tank 106 is sent to the ion-selective electrode group 102 via a sipper nozzle 102a by operations of the syringe (a sipper syringe) 108d and the valves 109h and 109i. At this time, a pinch valve 112 provided in a flow path between the ion-selective electrode group 102 and the reference electrode 103 is temporarily opened.

Similar to the diluted sample, an internal standard solution discharged to the dilution tank 106 is sent to the ion-selective electrode group 102 via the sipper nozzle 102a by operations of the syringe (a sipper syringe) 108d and the valves 109h and 109i.

A plurality of ion-selective electrodes constituting the ion-selective electrode group 102 are attached with ion sensitive membranes having a property in which an electromotive force changes according to a concentration of specific ions (for example, sodium ions (Na+), potassium ions (K+), or chlorine ions (Cl-)) in a sample solution. Accordingly, the ion-selective electrode group 102 outputs an electromotive force corresponding to each ion concentration in the sample solution.

When the pinch valve 112 is opened in a state where the diluted sample is sent to the ion-selective electrode group 102 and a reference electrode solution is sent to the reference electrode 103, the diluted sample and the reference electrode solution that are sent to a flow path are brought into contact with each other, and the ion-selective electrode group 102 and the reference electrode 103 are electrically conducted in the flow path.

A potential difference between each ion-selective electrode of the ion-selective electrode group 102 and the reference electrode 103 is measured by a voltage meter 113, and a measurement result (an electric signal) is sent to the control device 111 via an amplifier 114. Before or after measurement of the diluted sample, the internal standard solution is measured in a similar manner to the measurement of the diluted sample.

Although not shown, the control device 111 controls the entire operation of the electrolyte analyzer 100, and can be implemented by a computer including a calculation device such as a central processing unit (CPU), a storage device such as a random access memory (RAM) or a hard disk drive (HHD), and an input and output device such as an I/O port. The storage device and the input and output device are capable of exchanging data of the calculation device via an internal bus or the like. The input and output device is connected to each mechanism of the electrolyte analyzer 100, and the control device 111 controls an operation of each mechanism via the input and output device.

The control device 111 calculates a concentration of a specific electrolyte contained in the sample based on the potential difference between each ion-selective electrode of the ion-selective electrode group 102 and the reference electrode 103. An electrolyte concentration of the sample can be measured more accurately by performing calibration based on a measurement result of the internal standard solution. A specific method of calibration can be appropriately designed based on a known technique or the like by those skilled in the art. The control device 111 stores the calculated ion concentration for each ion in a storage unit (not shown) and outputs the ion concentration to an output device (for example, a display device).

The sample and the internal standard solution remained in the dilution tank 106 are aspirated by a waste liquid nozzle 115a by operations of a vacuum bin 115 and the valves 109j and 109k, and are sent to the waste liquid tank 110.

In the electrolyte analyzer 100 according to the present embodiment having the configuration described above, an ion concentration of the system water in the water supply tank 107 is measured by a water quality measuring unit 200 (the ion-selective electrode group 102, the reference electrode 103, and the voltage meter 113), and the control device 111 performs a water quality determination process of determining whether water quality of the system water is normal or abnormal based on a measurement result.

First, a principle of the water quality determination process according to the present embodiment will be described.

FIG. 2 is a diagram showing a relationship between a potential difference between an ion-selective electrode and a reference electrode, and a concentration of ions to be measured of the ion-selective electrode, when the system water (hereinafter, referred to as an ion solution) in which the ions to be measured are dissolved is measured by the electrolyte analyzer.

As shown in FIG. 2, for example, when the ion solution does not contain ions (hereinafter referred to as interference ions) that inhibit ion selectivity of the ion-selective electrode group 102, a concentration logarithm value (mmol/1) and a potential difference (mV) show a proportional relationship regardless of whether a concentration of the ions to be measured is high or low. On the other hand, when the ion solution contains a certain amount or more of interference ions, the relationship between the concentration logarithm value (mmol/1) and the potential difference (mV) deviates from the proportional relationship, and an inclination of the relationship between the concentration logarithm value (mmol/1) and the potential difference (mV) changes. The change in the inclination becomes more remarkable as the concentration of the interference ions increases. When the concentration of the ions to be measured is high, the inclination of the relationship between the concentration logarithm value (mmol/1) and the potential difference (mV) hardly changes regardless of the presence or absence of the interference ions (when the concentration of the ions is sufficiently lower than the concentration of the ions to be measured).

Therefore, the concentration of the interference ions contained in the system water is estimated by generating, using the system water, a plurality of types of ion solutions having a known concentration of the ions to be measured in each of a high concentration range and a low concentration range, measuring a potential difference between the ion-selective electrode group 102 and the reference electrode 103, and obtaining an inclination of a relationship between the concentration logarithm value (mmol/1) and the potential difference (mV). It is possible to obtain an inclination at a boundary between a concentration of the interference ions at which an influence on measurement accuracy of the electrolyte analyzer 100 is allowable and a concentration at which the influence is not allowable by experimentally obtaining an inclination of the relationship between the concentration logarithm value (mmol/1) and the potential difference (mV) by changing the concentration of the interference ions relative to the system water, and thus a water quality determination process of determining whether water quality of the system water is good (normal) or abnormal can be performed using the inclination as a threshold.

In the present embodiment, an example will be described in which chlorine ions (Cl-) are measured using, as an ion solution, a diluted internal standard solution prepared by diluting an internal standard solution with the system water. The water quality determination process is performed based on a result of measuring a plurality of types (for example, two types) of diluted internal standard solutions having known different concentrations in each of the high concentration range and the low concentration range.

Although details will be described by taking the measurement of the chlorine ions (Cl-) using the internal standard solution as an example in the present embodiment, the water quality determination process can be performed in a similar manner as long as the ion solution contains specific ions to be measured by the ion-selective electrode and has a known concentration, and a type of ions and a type of a reagent are not limited. In the electrolyte analyzer 100 shown in FIG. 1, the water quality determination process can be performed with sodium ions (Na+) or potassium ions (K+). In the case of including an ion-selective electrode for measuring magnesium ions (Mg+) or an ion-selective electrode for measuring lithium ions (Li+), the water quality determination process can also be performed using magnesium ions (Mg+ +) or lithium ions (Li+). Although a case where the diluted internal standard solution is used as the ion solution will be described in detail as an example in the present embodiment, a reference electrode solution or a calibrator used for calibration can also be used instead of the internal standard solution. Reagents having different known concentrations may be used, such as using an internal standard solution and a calibrator, in each of the low concentration range and the high concentration range.

Specifically, an internal standard solution in the internal standard solution bottle 104 is discharged to the dilution tank 106 via the internal standard solution nozzle 104a, and the system water in the water supply tank 107 is discharged to the dilution tank 106 via the system water nozzle 107a. A diluted internal standard solution is produced by diluting an internal standard solution having a known concentration with system water at a predetermined ratio. The diluted internal standard solution has two concentrations, for example, 80 (mmol/l) and 120 (mmol/1) in terms of a concentration of chlorine ions (C1-) in the high concentration range. A potential difference between the ion-selective electrode group 102 and the reference electrode 103 in each of the diluted internal standard solutions having two concentrations is measured by the voltage meter 113, and measurement results are plotted with a horizontal axis representing an electrolyte concentration (a logarithm) and a vertical axis representing a potential difference. A linear regression line is drawn between two points of the measurement results, and an inclination (a slope) of the linear regression line is obtained. The inclination is defined as an inclination (SL1) in the case of a high concentration.

The diluted internal standard solution in the low concentration range has two concentrations, for example, 8 (mmol/1) and 12 (mmol/1) in terms of a concentration of chlorine ions (C1-). In a similar manner to the high concentration range, a potential difference between the ion-selective electrode group 102 and the reference electrode 103 in each of the diluted internal standard solutions having two concentrations is measured by the voltage meter 113, and measurement results are plotted with a horizontal axis representing an electrolyte concentration (a logarithm) and a vertical axis representing a potential difference. A linear regression line is drawn between two points of the measurement results, and an inclination (a slope) of the linear regression line is obtained. The inclination is defined as an inclination (SL2) in the case of a low concentration.

For example, when a measurement lower limit value of each ion-selective electrode of the ion-selective electrode group 102 is sufficiently lower than 0.2 (mmol/1) and the system water does not contain the interference ions, the slope SL1 obtained for the diluted internal standard solution in the high concentration range and the slope SL2 obtained for the diluted internal standard solution in the low concentration range show the same value. Therefore, a ratio of the slope SL2 to the slope SL1, the slope SL1 as a criteria, is (SL2/SL1 $\times$ 100) = 100 (%). On the other hand, when the system water contains the interference ions, measurement sensitivity in the low concentration range is lowered due to an influence of the interference ions, and thus the ratio (SL2/SL1 $\times$ 100) of the slope SL2 relative to the slope SL1 is lower than 100 (%). For example, in a case where a threshold of a concentration of the interference ions set in consideration of the influence of the interference ions on measurement accuracy is 1.0 (mmol/l), when the ratio (SL2/SL1 $\times$ 100) of the slope SL2 to the slope SL1, the slope SL1 as a criteria, is 92 (%) or less, it can be determined that the influence on the measurement accuracy is not allowable, that is, water quality of the system water is abnormal.

Although specific concentrations of the chlorine ions (Cl-) are described in detail as an example, a concentration is not limited to the above concentrations, and an inclination may be obtained in each of the high concentration range and the low concentration range. Through studies, it was found that a concentration range of the high concentration range is preferably about 10 times or more a concentration range of the low concentration range. The larger a concentration difference between the high concentration range and the low concentration range, the more remarkable a difference in inclinations appears, and water quality abnormality can be determined with high accuracy.

A more appropriate concentration range is different depending on an ion type, when the water quality determination process is performed using chlorine ions (Cl-), the concentration range in the high concentration range is preferably 80 (mmol/1) to 120 (mmol/1) and the concentration range in the low concentration range is preferably 8 (mmol/1) to 12 (mmol/1), when the water quality determination process is performed using potassium ions (K+), the concentration range in the high concentration range is preferably 3 (mmol/1) to 7 (mmol/1) and the concentration range in the low concentration range is preferably 0.3 (mmol/1) to 0.7 (mmol/l), and when the water quality determination process is performed using sodium ions (Na+), the concentration range in the high concentration range is preferably 120 (mmol/1) to 160 (mmol/1) and the concentration range in the low concentration range is preferably 12 (mmol/1) to 16 (mmol/1). Although the accuracy of the water quality determination process can be improved by performing the water quality determination process in the above-described concentration ranges, the water quality determination process can also be performed in a concentration range other than the above-described concentration ranges.

The slopes SL1 and SL2 described above can be obtained according to the following (Formula 1).

$$SL = (EMFH - EMFL)/(LogCH - LogCL) \quad (Formula\ 1)$$

In Formula 1, EMFH represents a measured electromotive force of a high-concentration diluted internal standard solution, EMFL represents a measured electromotive force of a low-concentration diluted internal standard solution, CH represents a specified concentration value of the high-concentration diluted internal standard solution, and CL represents a specified concentration value of the low-concentration diluted internal standard solution. The specified concentration value of the high-concentration diluted internal standard solution and the specified concentration value of the low-concentration diluted internal standard solution are high-concentration or low-concentration specified concentration values in each concentration range.

A concentration of the internal standard solution is obtained according to the following (Formula 2) and (Formula 3).

$$CIS = CL \times 10a \quad (Formula\ 2)$$

$$a = (EMFS - EMFL)/SL \quad (Formula\ 3)$$

CIS represents a concentration of the internal standard solution, and EMFIS represents an electromotive force of the internal standard solution.

A concentration of a sample is calculated according to the following Formula (4).

$$E = E0 + (2.303\ RT/zF) \cdot logCS \quad (Formula\ 4)$$

CS represents a concentration of a sample, E represents a potential difference (mV) generated between an ion-selective electrode and a reference electrode, E0 represents a reference potential (mV), R represents a gas constant (8.3144 J-mol - 1-K - 1), T represents an absolute temperature (273.15+°C), n represents an ion valence, and F represents a Faraday constant (96.485 C-mol).

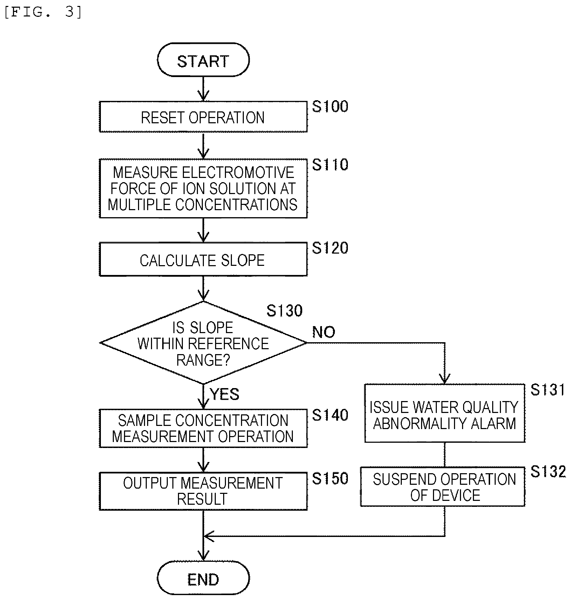

FIG. 3 is a flowchart showing the water quality determination process.

In FIG. 3, when the control device 111 is instructed to start analysis, the control device 111 first performs a reset operation (step S100).

Subsequently, diluted internal standard solutions having multiple concentrations are generated and measured by the water quality measuring unit 200 (the ion-selective electrode group 102, the reference electrode 103, and the voltage meter 113), and electromotive forces (potential differences) are measured (step S110).

Subsequently, the slopes SL1 and SL2 are respectively calculated based on measurement results of a diluted internal standard solution in a high concentration range and a diluted internal standard solution in a low concentration range (step S120), and it is determined whether the slope SL2 is within a reference range (step S130). Here, whether the slope SL2 is within the reference range refers to, for example, whether the ratio (SL2/SL1 $\times$ 100) of the slope SL2 to the slope SL1 is 92 (%) or more as described above.

When the determination result in step S130 is YES, it is determined that water quality of the system water is normal (good), a concentration measurement operation of a sample is performed (step S140), a measurement result is output (step S150), and the process ends. At this time, since the water quality of the system water is ensured, the measurement result can be obtained with high accuracy.

When the determination result in step S130 is NO, it is determined that the water quality of the system water is abnormal, a water quality abnormality alarm is issued (step S131), an operation of the device is stopped (step S132), and the process ends. At this time, since it is detected that the water quality of the system water is abnormal and measurement of a sample is not performed, it is possible to prevent measurement with low accuracy (low reliability) from being performed, and it is possible to prompt rapid improvement and restoration of an abnormal state by notifying an operator of the abnormality.

Although the water quality determination process is performed when the analysis is started in the flowchart shown in FIG. 3, the water quality determination process can be performed at any timing.

Effects of the present embodiment having the configuration described above will be described.

In the related art in which the silver nitrate solution (AgNO3) is used as a diluent, wastewater treatment is indispensable in consideration of an environmental load, and the wastewater treatment causes an increase in treatment cost and treatment time. In particular in a large hospital, a clinical examination center, or the like that performs a large number of measurement processes, since a usage amount of a diluent and an amount of wastewater are large, a burden of running cost or the like is enormous. In the related art in which a diluent is produced by mixing a diluent stock solution and pure water, since the pure water is not standardized, an error may occur in a measurement result of an electrolyte analyzer due to unspecified ions contained in the pure water.

On the other hand, in the present embodiment, since the electrolyte analyzer that measures an ion concentration in a liquid includes an electrolyte analysis unit including an ion-selective electrode used for measuring specific ions, and a reference electrode, a diluent supply unit that supplies a diluent for diluting a reagent or a sample, and a control device, and the control device measures each of diluted reagents with known specific ion concentrations at multiple different concentrations generated by diluting the reagents having known concentrations with a diluent, and performs a water quality determination process of the diluent based on an inclination obtained based on a measurement result, a burden related to measurement can be reduced while preventing a decrease in accuracy of the measurement result.

<Second Embodiment>

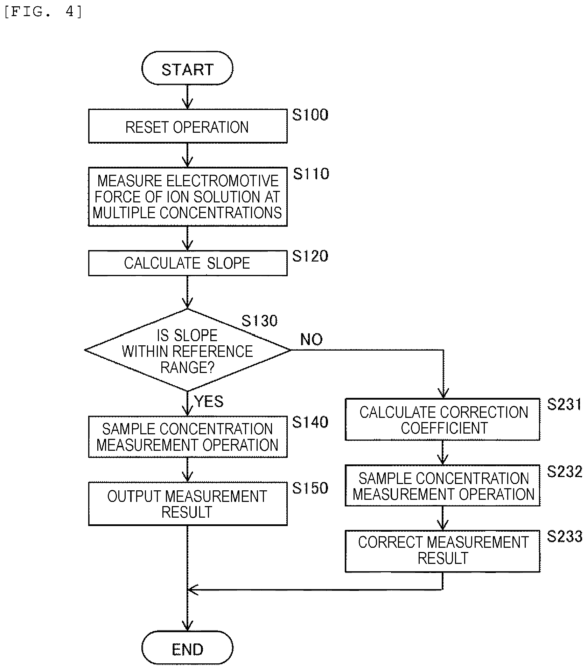

A second embodiment of the invention will be described with reference to FIG. 4.

In the present embodiment, an example will be described in which a correction coefficient is calculated instead of issuing an alarm when a concentration of the interference ions is not within the reference range. Other configurations are the same as those in the first embodiment, and only differences from the first embodiment will be described.

FIG. 4 is a flowchart showing a water quality determination process according to the present embodiment. Since processes when a slope is within the reference range (that is, the processes in steps S100 to S150) are the same as that in the first embodiment, only the processes in steps S231 to S232 will be described in the present embodiment.

In FIG. 4, when the determination result in step S130 is NO, it is determined that the water quality of the system water is abnormal, a correction coefficient is calculated (step S231), and a concentration measurement operation of a sample is performed (step S232). A measurement result of the sample is corrected by applying the correction coefficient to the measurement result of the sample (step S233), and the process is ended.

Here, the correction coefficient is used to correct a change in the measurement result of the sample due to an influence of the interference ions, and for example, a change amount of the measurement result of the sample relative to the concentration of the interference ions is experimentally obtained in advance, and a correction coefficient corresponding to a measured concentration of the interference ions is applied to the measurement result of the sample, thereby obtaining the measurement result with higher accuracy. That is, even when it is detected that the water quality of the system water is abnormal, since the measurement result of the sample is corrected according to the concentration of the interference ions, measurement can be performed with high accuracy without stopping a measurement operation of the sample.

In the present embodiment having the configuration described above, the same effect as that in the first embodiment can also be attained. Even when it is detected that the water quality is abnormal, the measurement can be continuously performed.

<Third Embodiment>

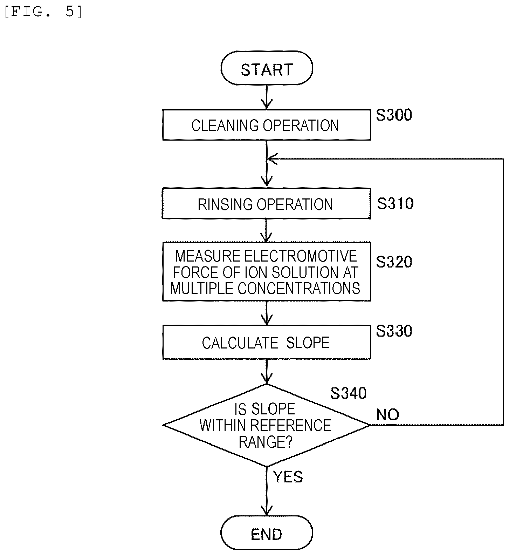

A third embodiment of the invention will be described with reference to FIG. 5.

In the present embodiment, a water quality determination process after cleaning the water supply tank 107 will be described.

In order to keep the system water normal, a user periodically cleans the water supply tank 107 with detergent. The detergent is, for example, a sodium hypochlorite containing chlorine ions (C1-) or sodium ions (Na+), or a chlorine dioxide containing chlorine ions (C1-). When rinsing after cleaning is not sufficient, ions in the detergent may act as interference ions and affect a measurement result of a sample. In the present embodiment, a water quality determination process is performed after the water supply tank 107 is cleaned.

FIG. 5 is a flowchart showing a cleaning treatment according to the present embodiment.

In FIG. 5, when cleaning is instructed, first, the detergent is put into the water supply tank 107 to clean the water supply tank (step S300), and to rinse the water supply tank, system water is put into the water supply tank 107 in which the detergent is input (step S310). Subsequently, in order to check whether the detergent remains in the water supply tank after the rinsing operation, electromotive forces of ion solutions at multiple concentrations are measured (step S320), and a slope is generated based on measurement results (step S330). Subsequently, it is determined whether the slope SL2 is within a reference range (step S340). Since the processes in steps S320 to S330 are the same as steps S110 to S130 in the first embodiment, detailed description thereof will be omitted.

When the determination result in step S340 is YES, it is determined that no detergent remains (rinsing is sufficient), and the process ends. When the determination result in the step S340 is NO, it is determined that the rinsing is insufficient and the detergent remains, the process returns to the step S310, and the rinsing operation is repeatedly performed by repeating the processes in steps S310 to S330 until the determination result is YES.

In the present embodiment, since whether rinsing is sufficient is detected by detecting a concentration of ions contained in the detergent, an ion solution needs to contain at least ions contained in the detergent and ions to be measured of the ion-selective electrode.

In the present embodiment, since it is possible to confirm that the detergent does not remain after the cleaning treatment of the water supply tank 107, it is possible to prevent the detergent from affecting a measurement result of a sample.

Although the example is described above in which the cleaning operation and the rinsing operation are automatically performed, the cleaning operation and the rinsing operation may be manually performed by a user. When the user manually performs the operations, the user gives an instruction to perform the water quality determination process to perform steps S320 to S330, the user is notified of whether the detergent remains, and when it is determined in step S340 that the slope SL2 is outside the reference range and the detergent remains, it is possible to prompt the user to add a rinsing operation.

<Other Embodiments>

The invention is not limited to the embodiments described above, and includes various modifications and combinations within a range not departing from the gist of the invention.

For example, although the water quality determination process is performed in which the SL1 and SL2 are obtained by measuring an ion solution in the high concentration range and an ion solution in the low concentration range in the first to third embodiments, the ion solution in the high concentration range may not be measured. A theoretical value may be obtained instead of the SL1. Since the theoretical value is different from an actual measured value, the accuracy of the water quality determination is lowered as compared with a case where the ion solution is measured and the SL1 is obtained, but the inclination when no interference ion is contained can be calculated using a theoretical value, and the reagent used in the water quality determination process can be saved. Although the high-concentration ion solution and the low-concentration ion solution are measured in each of the high concentration range and the low concentration range in the embodiments described above, the SL1 and SL2 may be respectively obtained using ion solutions of the same concentration for the low-concentration ion solution in the high concentration range and the high-concentration ion solution in the low concentration range. As described above, the water quality determination can be performed with high accuracy when there is a difference of about ten times in a concentration range between the high concentration range and the low concentration range, but if the water quality determination can be performed when the inclination between the low concentration range and the high concentration range can be reduced, the reagent used in the water quality determination process can be saved.

<Appendix>

The invention is not limited to the embodiments described above, and includes various modifications and combinations without departing from the gist of the invention. Further, the invention is not limited to the invention including all configurations described in the above embodiments, and includes a configuration in which a part of the configurations is deleted. Some or all of configurations, functions, and the like described above may be implemented by, for example, designing with an integrated circuit. In addition, the configurations, functions, and the like described above may be implemented by software by a processor interpreting and executing a program for implementing each function.

Reference Signs List

| 100, 100A, 100B: | electrolyte analyzer, |
| 101: | sample dispensing device, |
| 101a: | sample probe, |
| 101b: | sample container, |
| 102: | ion-selective electrode group, |
| 102a: | sipper nozzle, |
| 103: | reference electrode, |
| 104: | internal standard solution bottle, |
| 104a: | internal standard solution nozzle, |
| 105: | reference electrode solution bottle, |
| 106: | dilution tank, |
| 107: | water supply tank, |
| 107a: | system water nozzle, |
| 108a: | system water syringe, |
| 108b: | internal standard solution syringe, |
| 108c: | reference electrode solution syringe, |
| 108d: | sipper syringe, |
| 109a-109k: | valve, |
| 110: | waste liquid tank, |
| 111: | control device, |
| 112: | pinch valve, |
| 113: | voltage meter, |
| 114: | amplifier, |
| 115: | vacuum bin, |
| 115a: | waste liquid nozzle, |
| 116: | temperature controller, |
| 200: | water quality measuring unit |

**Claims**

1. An electrolyte analyzer that measures the ion concentration in a liquid, the analyzer comprising:

an electrolyte analysis unit including an ion-selective electrode used for measuring a specific ion and a reference electrode;
a diluent supply unit that supplies a diluent for diluting a reagent or a sample; and
a control device, wherein
the control device

measures each of the diluted reagents with known specific ion concentrations at multiple different concentrations generated by diluting the reagents at known concentrations with the diluent, and
performs a water quality determination process of the diluent based on the inclination obtained from the measurement results.

2. The electrolyte analyzer according to claim 1, wherein
the reagent having the known concentration is an internal standard solution, a calibrator, or a reference electrode solution.

3. The electrolyte analyzer according to claim 1, wherein
the control device measures the diluted reagents having four different concentrations and performs a water quality determination process of the diluent based on the inclination obtained from the measurement results of the two concentrations in the high concentration range and the inclination obtained from the measurement results of the two concentrations in the low concentration range.

4. The electrolyte analyzer according to claim 3, wherein
the concentration in the high concentration range is 10 times or more the concentration in the low concentration range.

5. The electrolyte analyzer according to claim 4, wherein

as the specific ion, the concentration range in the low concentration range for chlorine ion (Cl-) is 8 to 12 (mmol/l),
the concentration range in the low concentration range for potassium ion (K+) is 0.3 to 0.7 (mmol/1), and
the concentration range in the low concentration range for sodium ion (Na+) is 12 to 16 (mmol/1).

6. The electrolyte analyzer according to claim 1 or 2, wherein
a water quality determination process of the diluent is performed based on the inclination obtained from the results of measuring at multiple concentrations the diluted reagents having ion concentrations selected from the range of 8-12 (mmol/1) in the case of chlorine ion (Cl-), 0.3 to 0.7 (mmol/1) in the case of potassium ion (K+), and 12 to 16 (mmol/1) in the case of sodium ion (Na+) as the specific ion.

7. The electrolyte analyzer according to any one of claims 1 to 6, wherein
if the water quality of the diluent is determined to be abnormal in the water quality determination process, the control device corrects the measurement result of the sample by the ion-selective electrode by using a predetermined correction coefficient according to the ion concentration of the diluent.

8. The electrolyte analyzer according to any one of claims 1 to 6, wherein
an alarm is issued when the water quality of the diluent is determined to be abnormal in the water quality determination process.

9. The electrolyte analyzer according to any one of claims 1 to 6, further comprising:

a water supply tank for storing the diluent, wherein
if the water quality of the diluent is determined to be abnormal in the water quality determination process after the cleaning step including the cleaning of the water supply tank with a detergent and the rinsing, it is determined that the detergent remains.

10. The electrolyte analyzer according to any one of claims 1 to 8, wherein
the diluent is system water.

11. An analysis method that measures the ion concentration of a specific ion in a liquid based on the potential difference between an ion-selective electrode and a reference electrode, the method comprising:
performing a water quality determination process of a diluent based on the inclination obtained from the measurement results of each of the diluted reagents with known specific ion concentrations at multiple concentrations generated by diluting the reagents with known concentrations with the diluent for diluting a reagent or a sample.

12. The analysis method according to claim 11, wherein
a water quality determination process of the diluent is performed based on the inclination obtained from the measurement results of the two concentrations in the high concentration range and the inclination obtained from the measurement results of the two concentrations in the low concentration range, among the measurement results obtained by measuring the diluted reagents having four different concentrations.

13. The analysis method according to claim 11, wherein
a water quality determination process of the diluent is performed based on the inclination obtained from the results of measuring at multiple concentrations the diluted reagents having the ion concentration selected from the range of 8 to 12 (mmol/1) in the case of chlorine ion (C1-), 0.3 to 0.7 (mmol/1) in the case of potassium ion (K+), and 12 to 16 (mmol/1) in the case of sodium ion (Na+) as the specific ion.

14. The analysis method according to any one of claims 11 to 13, wherein
if the water quality of the diluting wall is determined to be abnormal in the water quality determination process, a predetermined correction coefficient is used according to the ion concentration of the diluent to correct the measurement result of the sample by the ion-selective electrode.

15. The analysis method according to any one of claims 11 to 13, comprising:

a cleaning step including a cleaning treatment using a detergent for a water supply tank for storing a diluent and a rinsing treatment, wherein
if the water quality of the diluent is determined to be abnormal in the water quality determination process after the cleaning step, it is determined that the detergent remains.

[FIG. 1]

[FIG. 2]

[FIG. 3]

```
                    ┌─────────────┐
                    │    START    │
                    └─────────────┘
                           │
                           ▼
              ┌──────────────────────────┐ S100
              │      RESET OPERATION      │
              └──────────────────────────┘
                           │
                           ▼
              ┌──────────────────────────┐ S110
              │    MEASURE ELECTROMOTIVE  │
              │  FORCE OF ION SOLUTION AT │
              │  MULTIPLE CONCENTRATIONS  │
              └──────────────────────────┘
                           │
                           ▼
              ┌──────────────────────────┐ S120
              │      CALCULATE SLOPE      │
              └──────────────────────────┘
                           │
                           ▼        S130
                        ◇─────────◇
                       IS SLOPE           NO
                   WITHIN REFERENCE ─────────────┐
                       RANGE?                    │
                        ◇─────────◇              │
                           │ YES                 ▼
                           ▼          ┌──────────────────────┐ S131
              ┌──────────────────────┐│  ISSUE WATER QUALITY │
              │ SAMPLE CONCENTRATION  │ S140 │ ABNORMALITY ALARM  │
              │ MEASUREMENT OPERATION │    └──────────────────────┘
              └──────────────────────┘              │
                           │                        ▼
                           ▼          ┌──────────────────────┐ S132
              ┌──────────────────────┐ S150 │ SUSPEND OPERATION  │
              │  OUTPUT MEASUREMENT   │    │      OF DEVICE      │
              │       RESULT          │    └──────────────────────┘
              └──────────────────────┘              │
                           │                        │
                           ▼◄───────────────────────┘
                    ┌─────────────┐
                    │     END     │
                    └─────────────┘
```

[FIG. 4]

```
                    ┌─────────┐
                    │  START  │
                    └────┬────┘
                         ↓
              ┌────────────────────┐
              │  RESET OPERATION   │ S100
              └─────────┬──────────┘
                        ↓
              ┌────────────────────┐
              │MEASURE ELECTROMOTIVE│ S110
              │FORCE OF ION SOLUTION AT
              │MULTIPLE CONCENTRATIONS
              └─────────┬──────────┘
                        ↓
              ┌────────────────────┐
              │  CALCULATE  SLOPE  │ S120
              └─────────┬──────────┘
                        ↓
                    S130
                  ◇ IS SLOPE ◇         NO
              < WITHIN REFERENCE >──────────────┐
                  ◇  RANGE?  ◇                  │
                        │ YES                   ↓
                        ↓              ┌────────────────────┐
              ┌────────────────────┐   │CALCULATE CORRECTION│ S231
              │SAMPLE CONCENTRATION│   │    COEFFICIENT     │
              │MEASUREMENT OPERATION│S140└─────────┬──────────┘
              └─────────┬──────────┘             ↓
                        ↓              ┌────────────────────┐
              ┌────────────────────┐   │SAMPLE CONCENTRATION│ S232
              │ OUTPUT MEASUREMENT │S150│MEASUREMENT OPERATION│
              │       RESULT       │   └─────────┬──────────┘
              └─────────┬──────────┘             ↓
                        │              ┌────────────────────┐
                        │              │ CORRECT MEASUREMENT│ S233
                        │              │       RESULT       │
                        │              └─────────┬──────────┘
                        ↓←───────────────────────┘
                    ┌─────────┐
                    │   END   │
                    └─────────┘
```

[FIG. 5]

```
                    ( START )
                        |
                        v
        +-----------------------------+  S300
        |     CLEANING OPERATION      |
        +-----------------------------+
                        |
                        v  <-----------------------------+
        +-----------------------------+  S310            |
        |      RINSING OPERATION      |                  |
        +-----------------------------+                  |
                        |                                |
                        v                                |
        +-----------------------------+  S320            |
        |   MEASURE ELECTROMOTIVE     |                  |
        |  FORCE OF ION SOLUTION AT   |                  |
        |   MULTIPLE CONCENTRATIONS   |                  |
        +-----------------------------+                  |
                        |                                |
                        v                                |
        +-----------------------------+  S330            |
        |       CALCULATE SLOPE       |                  |
        +-----------------------------+                  |
                        |                                |
                        v         S340                   |
                    /         \        NO                |
                  /  IS SLOPE   \------------------------+
                 < WITHIN REFERENCE >
                  \   RANGE?   /
                    \        /
                        |
                       YES
                        |
                        v
                    ( END )
```

| | INTERNATIONAL SEARCH REPORT | International application No. |
|---|---|---|
| | | **PCT/JP2022/022023** |

**A. CLASSIFICATION OF SUBJECT MATTER**

*G01N 27/416*(2006.01)i
FI: G01N27/416 356; G01N27/416 351B; G01N27/416 351K

According to International Patent Classification (IPC) or to both national classification and IPC

**B. FIELDS SEARCHED**

Minimum documentation searched (classification system followed by classification symbols)

G01N27/416

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); Scopus

**C. DOCUMENTS CONSIDERED TO BE RELEVANT**

| Category* | Citation of document, with indication, where appropriate, of the relevant passages | Relevant to claim No. |
|---|---|---|
| X | JP 2011-122823 A (HITACHI HIGH-TECHNOLOGIES CORP) 23 June 2011 (2011-06-23) paragraphs [0001], [0013], [0017]-[0020], [0025]-[0026], fig. 1 | 1, 6, 8, 11, 13 |
| Y | paragraphs [0001], [0013], [0017]-[0020], [0025]-[0026], fig. 1 | 2, 7, 9-10, 14-15 |
| A | entire text, all drawings | 3-5, 12 |
| Y | JP 8-220050 A (HITACHI LTD) 30 August 1996 (1996-08-30) paragraphs [0014]-[0015] | 2 |
| Y | JP 4-291147 A (HITACHI LTD) 15 October 1992 (1992-10-15) paragraphs [0004], [0006]-[0007] | 7, 14 |
| Y | JP 2001-4586 A (HITACHI LTD) 12 January 2001 (2001-01-12) paragraph [0036] | 9, 15 |
| Y | JP 10-62375 A (SHIMADZU CORP) 06 March 1998 (1998-03-06) paragraphs [0011], [0026]-[0027], [0034], [0036] | 10 |

☑ Further documents are listed in the continuation of Box C.  ☑ See patent family annex.

| | |
|---|---|
| \* Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **27 June 2022** | **12 July 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**

International application No.

**PCT/JP2022/022023**

## C. DOCUMENTS CONSIDERED TO BE RELEVANT

| Category* | Citation of document, with indication, where appropriate, of the relevant passages | Relevant to claim No. |
|---|---|---|
| A | YSI. Primer: Ion selective measurement in online analysis. catalog. ba76001e01. YSI. 2012, pp. 1-10, 22, [online], [retrieval date 27 June 2022], Internet:<URL: https://www.ysi.com/File%20Library/Documents/Manuals/ba76001-Online-ISE-Primer-e01.pdf> p. 8, 4th paragraph to p. 9, 1st paragraph | 3, 12 |
| A | JP 2001-264283 A (HITACHI LTD) 26 September 2001 (2001-09-26) entire text, all drawings | 1-15 |
| P, X | WO 2021/161704 A1 (HITACHI HIGH TECH CORP) 19 August 2021 (2021-08-19) claims 1-5, 8-10, paragraphs [0035]-[0036] | 1-8, 10-14 |
| P, A | entire text, all drawings | 9, 15 |

Form PCT/ISA/210 (second sheet) (January 2015)

**INTERNATIONAL SEARCH REPORT**
Information on patent family members

International application No.

**PCT/JP2022/022023**

| Patent document cited in search report | | | Publication date (day/month/year) | Patent family member(s) | | | Publication date (day/month/year) |
|---|---|---|---|---|---|---|---|
| JP | 2011-122823 | A | 23 June 2011 | US 2012/0261260 A1 paragraphs [0001], [0023], [0023]-[0036], [0049]-[0050], fig. 1 WO 2011/070719 A1 EP 2511699 A1 CN 102648407 A | | | |
| JP | 8-220050 | A | 30 August 1996 | (Family: none) | | | |
| JP | 4-291147 | A | 15 October 1992 | (Family: none) | | | |
| JP | 2001-4586 | A | 12 January 2001 | (Family: none) | | | |
| JP | 10-62375 | A | 06 March 1998 | (Family: none) | | | |
| JP | 2001-264283 | A | 26 September 2001 | (Family: none) | | | |
| WO | 2021/161704 | A1 | 19 August 2021 | (Family: none) | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H8211016 A

- JP 2018017543 A